# EUROPÄISCHE PATENTSCHRIFT

(11) **EP 1 212 983 B1**
(45) Veröffentlichungstag und Bekanntmachung des Hinweises auf die Patenterteilung: **06.12.2006**
(21) Anmeldenummer: 01128904.8
(22) Anmeldetag: 05.12.2001
(51) Int. Cl.: A61B 17/28, A61B 17/32

(54) **Chirurgisches Instrument**
Surgical device
Instrument chirurgical

(30) Priorität: 08.12.2000 DE 10061512
(43) Veröffentlichungstag der Anmeldung: 12.06.2002
(73) Patentinhaber: Tontarra Medizintechnik GmbH, 78573 Wurmlingen (DE)
(72) Erfinder: Tontarra, Thomas, 78573 Wurmlingen (DE)
(74) Vertreter: Mammel, Ulrike

(56) Entgegenhaltungen:
- WO-A-00/01312
- DE-A- 4 316 768
- DE-U- 29 718 969
- DE-U- 29 922 271
- US-A- 6 126 674

## Beschreibung

Die Erfindung betrifft ein chirurgisches Instrument gemäß dem Oberbegriff des Anspruchs 1.

Derartige chirurgische Instrumente sind beispielsweise durch eine Firma Jarit bekannt. Bei diesen chirurgischen Instrumenten handelt es sich um Stanzen, welche eingesetzt werden, um bei chirurgischen Eingriffen Gewebe, Knochen oder dergleichen zu entfernen. Diese Vorrichtungen weisen ein Hauptteil und zumindest ein relativ dazu bewegbares Teil, einen sogenannten Schieber, auf sowie einen Handgriff, der einen mit dem Hauptteil verbundenen feststehenden und einen mit dem bewegbaren verbundenen Teil betätigbaren Griff aufweist. Durch Öffnen und Schließen des Griffes wird der bewegbare Teil zum Hauptteil geschlossen und geöffnet, wobei während der Schließbewegung beispielsweise das Entfernen von Gewebe, Knochen oder dergleichen ermöglicht ist.

Derartige chirurgische Instrumente müssen nach jedem chirurgischen Eingriff gereinigt und sterilisiert werden, um die Übertragung von Infektionen oder dergleichen bei weiteren Eingriffen zu vermeiden. Diese Instrumente weisen jedoch den Nachteil auf, daß das bewegbare Teil nicht von dem Hauptteil lösbar ist, so daß sich im Bereich von Führungen zwischen dem bewegbaren und dem Hauptteil Bakterien ansammeln können.

Aus der DE 4316768 A1 ist ein chirurgisches Instrument bekannt, bei welchen für die Reinigung und Sterilisation das bewegbare Teil von dem Hauptteil vollständig gelöst und entfernt werden kann. Das Hauptteil und das bewegbare Teil sind dabei jeweils mit Kennzeichnungen versehen, so dass nach dem Reinigen und Sterilisieren der Instrumente eine Zuordnung des bewegbaren Teils zum Hauptteil ermöglicht ist. Dies ist erforderlich, da die chirurgischen Instrumente im zusammengebauten Zustand durch Schleifen endbearbeitet werden, um einen absatzfreien Übergang zwischen dem Hauptteil und dem bewegbaren Teil zu ermöglichen. Derartige Instrumente weisen den Nachteil auf, dass der Handgriff ebenfalls zumindest teilweise von dem Hauptteil zu lösen ist um das bewegbare Teil zumindest teilweise von dem Hauptteil zu entfernen. Dadurch wird auch durch die Koordination der Bewegungsabläufe von mehreren Bauteilen für den Zusammenbau erschwert. Die aus den Druckschriften WO 00/01312 A, US 6 126 674 A, DE 299 22 271 U und DE 297 18 969 U bekannten chirurgischen Instrumente weisen die selben Nachteile auf.

Der Erfindung liegt deshalb die Aufgabe zugrunde, ein chirurgisches Instrument zu schaffen, welches zum Reinigen und Sterilisieren ein Lösen des bewegbaren Teils zum Hauptteil ermöglicht, ohne dass eine vollständige Trennung des bewegbaren Teils zum Hauptteil gegeben ist und welches bedarfsmäßig in einfacher Weise eine vollständige Abnahme des bewegbaren Teils zum Hauptteil vorsieht.

Diese Aufgabe wird erfindungsgemäß durch die Merkmale des Anspruchs 1 gelöst.

Durch die erfindungsgemäße Ausgestaltung des chirurgischen Instruments ist vorgesehen, dass das bewegbare Teil und der betätigbare Griff durch eine lösbare Gelenkverbindung in einer ersten Phase schwenkbar und in einer zweiten Phase zerlegbar zueinander angeordnet sind. In einer ersten Phase kann eine einfache und sichere Handhabung zur Reinigung und Sterilisation gegeben sein. Das bewegbare Teil bleibt mit dem Hauptteil verbunden, jedoch in einer Anordnung, welche die hygienischen Anforderungen erfüllt. In einer zweiten Phase kann das bewegbare Teil von dem betätigbaren Griff aufgrund der lösbaren Gelenkverbindung vollständig gelöst werden. Dadurch kann eine vollständig getrennte Reinigung der Teile erfolgen. Ebenso kann ein leichter Austausch des bewegbaren Teils gegeben sein. Durch diese konstruktive Ausgestaltung kann eine einfache Handhabung gegeben sein, die eine schnelle und einfache Abnahme des bewegbaren Teils vom Hauptteil für eine Reinigung und Desinfektion sowie ein anschließender Zusammenbau für den chirurgischen Einsatz ermöglicht.

Darüber hinaus ist durch die Ausgestaltung der Verriegelungsvorrichtung ein chirurgisches Instrument geschaffen, welches in der Handhabung den bisherigen Instrumenten entspricht, so dass keine Umgewöhnung für den Operateur erforderlich ist. Derartige chirurgische Instrumente können für sämtliche medizinische Bereiche einsetzbar sein.

Nach einer vorteilhaften Ausgestaltung der Erfindung ist vorgesehen, dass die lösbare Gelenkverbindung durch eine vorzugsweise U-förmige Ausnehmung in dem betätigbaren Handgriff und einen in der Ausnehmung gelagerten Stift des bewegbaren Teils ausgebildet ist und das offene Ende der Ausnehmung durch ein federnd nachgiebiges Rastelement verengt ist. Dadurch kann nach dem Lösen des bewegbaren Teils aus der Führung des Hauptteils das bewegbare Teil zunächst mit dem betätigbaren Handgriff verbunden bleiben. Nachdem beispielsweise durch Schwenken das bewegbare Teil gegenüber dem Hauptteil teilweise abgehoben ist, kann bei einer weiteren Schwenkbewegung das zum feststehenden Handgriff weisende Ende des bewegbaren Teils an einem Abschnitt des Handgriffs angreifen und ein Gegenlager bilden, so dass der in der Ausnehmung gelagerte Stift an dem nachgiebigen Rastelement vorbeigeführt wird. Sofern die Schwenkbewegung des bewegbaren Teils nahe dem das Schneidelement aufnehmenden Ende durchgeführt wird, können aufgrund der Hebelkräfte geringe Kräfte genügen, damit das bewegbare Teil vollständig von dem Hauptteil lösbar ist. Alternativ kann auch vorgesehen sein, daß ein Abheben des bewegbaren Teils nach dem Freiwerden aus der Führung des Hauptteils derart erfolgt, daß ohne dem Anliegen des zum Handgriff weisenden Ende des bewegbaren Hauptteiles an dem Gegenlager ein vollständiges Lösen des bewegbaren Teils gegeben ist.

Nach einer vorteilhaften Weiterbildung der lösbaren Gelenkverbindung ist vorgesehen, daß das Rastelement als Kugel ausgebildet ist, welche durch eine Federkraft, die vorzugsweise einstellbar ist, die Ausnehmung verengt. Dadurch kann die Kraft für die Freigabe des bewegbaren Teils von dem betätigbaren Handgriff eingestellt werden.

Nach einer weiteren alternativen Ausgestaltung der lösbaren Gelenkverbindung ist vorgesehen, daß das offene Ende der U-förmigen Ausnehmung durch ein elastisch nachgiebiges Rastelement, vorzugsweise einem Elastomer, ausgebildet ist. Dadurch kann eine einfache Ausgestaltung vorgesehen sein, welche eine einmal voreingestellte beziehungsweise durch die Materialauswahl bestimmte Kraft die Freigabe des Stiftes des bewegbaren Teiles aus der vorzugsweise U-förmigen Ausnehmung des betätigbaren Handgriffes ermöglicht.

Nach einer weiteren vorteilhaften Ausgestaltung der Erfindung ist vorgesehen, daß beim Überführen des bewegten Teils nach dem Reinigen in eine Arbeitsposition zunächst ein Positionieren des bewegbaren Teils zum betätigbaren Handgriff erfolgt und in einem nachfolgenden Schritt die Führungsabschnitte des bewegbaren Teils in die Aufnahme des Hauptteils eingreift und durch Drücken des betätigbaren Griffs die Führungen selbsttätig ineinander greifen. Dadurch kann ein sicherer und zwangsweiser Zusammenbau erzielt werden, wobei das Drücken des betätigbaren Griffes soweit erfolgt, daß die Verriegelungsvorrichtung selbständig in die Verriegelungsposition oder manuell in diese überführbar ist, so daß im Anschluß daran der Arbeitshub für das bewegbare Teil freigegeben ist.

Nach einer weiteren vorteilhaften Ausgestaltung der Erfindung ist vorgesehen, daß die Führung im Hauptteil einen ersten schrägverlaufenden Abschnitt aufweist, welcher das bewegbare Teil auf das Hauptteil zubewegt und in eine Arbeitsposition überführt. Dadurch kann ein leichtes Zusammenführen und vollständiges Aneinanderliegen der Führungsflächen zwischen dem Hauptteil und bewegbaren Teil erzielt werden.

Nach einer weiteren vorteilhaften Ausgestaltung der Erfindung ist vorgesehen, daß der Riegel kraft- und/oder formschlüssig in einer ersten Verriegelungsposition anordenbar ist. Dadurch kann sichergestellt sein, daß während eines chirurgischen Eingriffes ein selbständiges Öffnen der Verriegelungsvorrichtung verhindert werden kann.

Nach einer vorteilhaften Ausgestaltung der Erfindung ist vorgesehen, daß die Verriegelungsvorrichtung einen Riegel aufweist, der an dem Handgriff vorgesehen ist. Dadurch kann eine einfache Einhandbetätigung für das Entriegeln der Verriegelungsvorrichtung vorgesehen sein. Der Riegel kann sowohl an dem feststehenden Handgriff als auch an dem betätigbaren Griff vorgesehen sein. Diese Anordnung kann in Abhängigkeit der Ausführungsform frei gewählt werden.

Nach einer weiteren vorteilhaften Ausgestaltung ist vorgesehen, daß in dem feststehenden Griff ein schwenkbarer Riegel vorgesehen ist, der nahe einer Schwenkachse an dem betätigbaren Griff angreift. Dadurch kann eine kompakte Bauweise erzielt werden, wobei vorteilhafterweise auf die bisherige Geometrie und Größe der chirurgischen Instrumente zurückgegriffen werden kann.

Nach einer weiteren vorteilhaften Ausgestaltung der Erfindung ist vorgesehen, daß der schwenkbare Riegel einen zu seiner Verriegelungsposition bestimmenden Anschlag aufweist. Dadurch kann der Arbeitshub des bewegbaren Teils begrenzt werden, wobei die Hubbegrenzung des bewegbaren Teils durch den Riegel zumeist die Ausgangsposition des bewegbaren Teils für einen Arbeitshub bestimmt.

Nach einer weiteren vorteilhaften Ausgestaltung der Erfindung ist vorgesehen, daß der Riegel in eine Entriegelungsposition schwenkbar ist und einen weiteren Schwenkbereich des betätigbaren Griffs freigibt, welcher vorzugsweise durch ein an dem Hauptteil vorgesehenen Anschlag begrenzt ist. Dadurch kann der bewegbare Teil relativ zum Hauptteil derart bewegt werden, daß die ineinandergreifenden Führungsabschnitte voneinander getrennt werden können, um das bewegbare Teil um die Gelenkverbindung zu schwenken und von dem Hauptteil zumindest teilweise abzuheben. Die Größe des Schwenkbereiches kann vorteilhafterweise in Abhängigkeit der Länge der ineinandergreifenden Führungen abgestimmt sein, so daß auch ein sehr kurzer Schwenkbereich genügen würde, um die ineinandergreifenden Führungen zwischen dem Hauptteil und dem bewegbaren Teil voneinander zu trennen.

Nach einer weiteren vorteilhaften Ausgestaltung der Erfindung ist vorgesehen, daß der Riegel an einem Abschnitt zwischen einer Schwenkachse des betätigbaren Handgriffs und der Gelenkverbindung angreift. Dadurch kann eine kompakte Bauweise geschaffen werden und der Riegel nahezu vollständig in dem feststehenden Handgriff integriert sein. Darüber hinaus ist diese Verriegelungsvorrichtung für die sonstige Handhabung nicht störend.

Nach einer weiteren vorteilhaften Ausgestaltung der Erfindung ist vorgesehen, daß der Riegel einen Verriegelungsabschnitt aufweist, der an einem komplementär ausgebildeten Schwenkabschnitt des betätigbaren Handgriffs angreift. Dadurch kann eine definierte Anlage des Schwenkabschnitts an dem Verriegelungsabschnitt gegeben sein. Vorteilhafterweise kann dadurch erzielt werden, daß durch die Ausbildung einer Art Hinterschneidung beziehungsweise einer Anlagefläche und einer sich anschließenden Rastnocke erzielt werden kann, daß nach jedem Arbeitshub bei Rückstellung des betätigbaren Griffs in eine Ausgangsposition der Riegel in eine Verriegelungsposition gedrückt wird. Sollte sich der Riegel aus seiner Verriegelungsposition auch nur geringfügig gelöst haben, wird dieser nach jeden Arbeitshub automatisch in diese wieder zurückgeführt. Dadurch kann eine sichere Handhabung gewährleistet sein. Die Anordnung des Riegels an dem feststehenden Handgriff hat des weiteren den Vorteil, daß zwischen dem feststehenden Handgriff und dem Hauptteil ein Übergangsbereich vorgesehen ist, der den Verriegelungsmechanismus nahezu vollständig abschließt, so daß diese Verriegelungsvorrichtung vor mechanischen Beschädigungen von außen gleichzeitig geschützt sein kann.

Weitere vorteilhafte Ausgestaltungen und Weiterbildungen der Erfindung sind in den weiteren Ansprüchen näher beschrieben.

Anhand der nachfolgenden Zeichnungen sind vorteilhafte Ausführungsformen näher beschrieben. Es zeigen:
- Figur 1: eine schematische Seitenansicht einer erfindungsgemäßen Ausführungsform in einer Ausgangsposition,
- Figur 2: eine schematische Seitenansicht der Ausführungsform gemäß Figur 1 in einer Arbeitsposition,
- Figur 3: eine schematische Seitenansicht der Ausführungsform gemäß Figur 1 in einer Zwischenposition mit einer entriegelten Verriegelungsvorrichtung,
- Figur 4: eine schematische Seitenansicht der Ausführungsform gemäß Figur 1 in einer ersten Phase,
- Figur 5: eine schematische Seitenansicht der Ausführungsform gemäß Figur 1 beim Übergang der ersten in eine zweite Phase,
- Figur 6: eine schematische Seitenansicht der Ausführungsform gemäß Figur 1, bei der das bewegbare Teil aus der lösbaren Gelenkverbindung gelöst ist,
- Figur 7: eine schematisch vergrößerte Ansicht der erfindungsgemäß lösbaren Gelenkverbindung der Ausführungsform in Figur 1,
- Figur 8: eine schematische Seitenansicht einer erfindungsgemäßen Ausführungsform mit einer alternativen Verriegelungsvorrichtung,
- Figur 9: eine schematische Seitenansicht einer erfindungsgemäßen Ausführungsform mit einer weiteren alternativen Verriegelungsvorrichtung und
- Figur 10: eine schematische Seitenansicht einer erfindungsgemäßen Ausführungsform mit einer weiteren alternativen Verriegelungsvorrichtung.

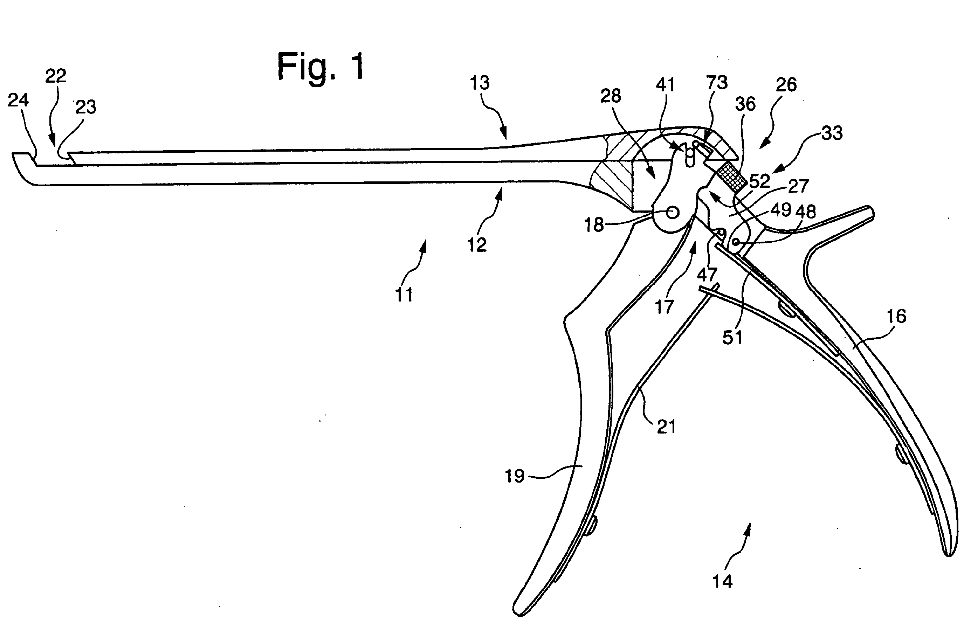

Eine erste erfindungsgemäße Ausführungsform eines chirurgischen Instrumentes 11 ist in den Figuren 1 bis 7 dargestellt. Bei diesem chirurgischen Instrument 11 handelt es sich beispielsweise um eine sogenannte Stanze, welche bei chirurgischen Eingriffen für die Entfernung von Gewebe, Knochen oder dergleichen eingesetzt wird. Die Erfindung ist nicht auf diese Stanzen beschränkt, sondern kann auf alle chirurgischen Instrumente übertragen werden, welche dieselbe Problematik hinsichtlich der Reinigung und Sterilisation sowie Zuordnung von Bauteilen aufweist.

Das chirurgische Instrument gemäß Figur 1 weist einen Hauptteil 12 auf, welches ein bewegbares Teil 13, das relativ zum Hauptteil 12 verschiebbar ist, aufnimmt. An dem Hauptteil 12 ist ein Handgriff 14 angeordnet. Das Hauptteil 12 geht in einen feststehenden Griff 16 des Handgriffs 14 über und weist im Übergangsbereich 17 vom Hauptteil zum feststehenden Griff 16 eine Drehachse 18 auf, um welche ein betätigbarer Griff 19 schwenkbar angeordnet ist. Der betätigbare Griff 19 und feststehende Griff 16 werden durch ein Federelement 21 in einer Ausgangsposition 22 angeordnet. In dieser Ausgangsposition 22 sind die Schneidelemente 23, 24 zueinander beabstandet, wobei dieser Abstand durch den maximalen Weg eines Arbeitshubes bestimmt ist. Diese Ausgangsposition 22 ist des weiteren durch eine Verriegelungsvorrichtung 26 bestimmt. Ein Riegel 27 der Verriegelungsvorrichtung 26 begrenzt die durch das Federelement 21 bewirkte Schwenkbewegung des betätigbaren Griffs 19 um die Drehachse 18. Dabei liegt ein kurzer Hebelabschnitt 28 an dem Riegel 27 an.

Bei Betätigung des Griffes 19 wird dieser um die Drehachse 18 geschwenkt, wobei das bewegbare Teil 13 gemäß der Darstellung nach links bewegt wird, um das Schneidelement 23 auf das Schneidelement 24 zuzubewegen.

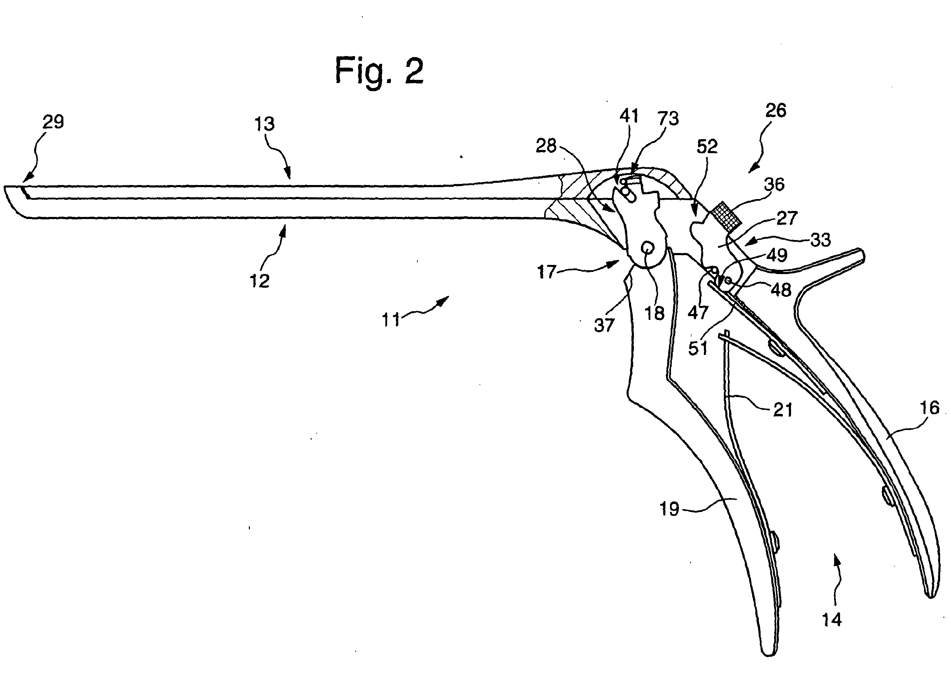

In Figur 2 ist der Arbeitshub beendet und das chirurgische Instrument 11 in eine Arbeitsposition 29 dargestellt. Das zu entfernende Gewebe oder der Knochen oder dergleichen ist in einem innerhalb den Schneidelementen 23, 24 vorgesehenen Hohlraum eingeschlossen und kann somit entfernt werden. Nachdem das chirurgische Instrument 11 aus dem Bereich des chirurgischen Eingriffs entfernt wurde, kann der Handgriff 14 gelöst werden, wodurch das chirurgische Instrument 11 sich selbständig durch die Federelemente 21 in einer Ausgangsposition 22 positioniert. Das zu entfernende Material kann sich dann selbständig von der Schneide 23 lösen, da vorteilhafterweise die Führungselemente 31, 32 als Auswerfer dienen.

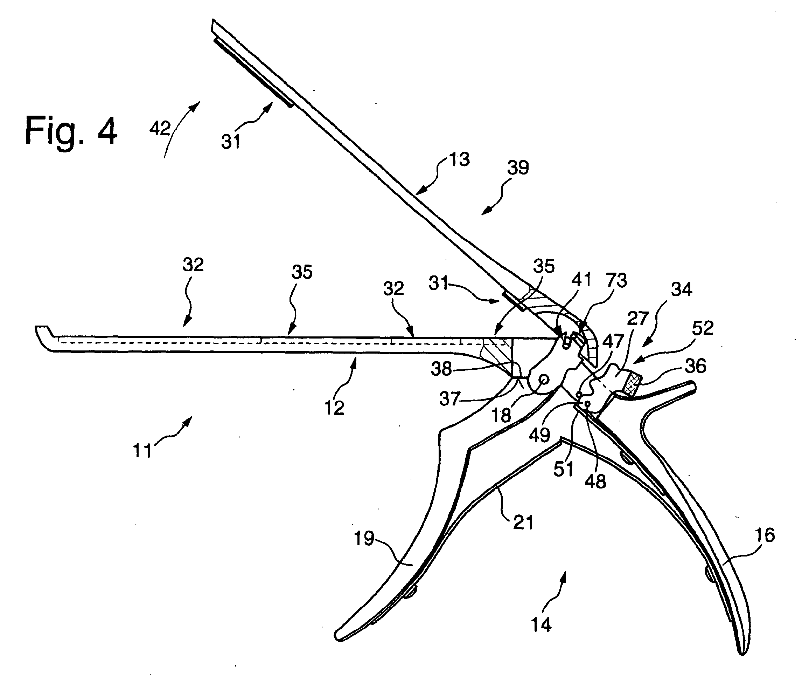

In Figur 4 ist beispielsweise das Führungselement 31 an dem beweglichen Teil 13 dargestellt. Diese vorteilhafterweise T-förmig ausgebildete Feder greift in eine entsprechende Nut, die das Führungselement 32 bildet, im Hauptteil 12 ein. In Abhängigkeit der Ausbildung des chirurgischen Instruments 11 können auch die Führungselemente 31, 32 angepaßt und ausgebildet sein.

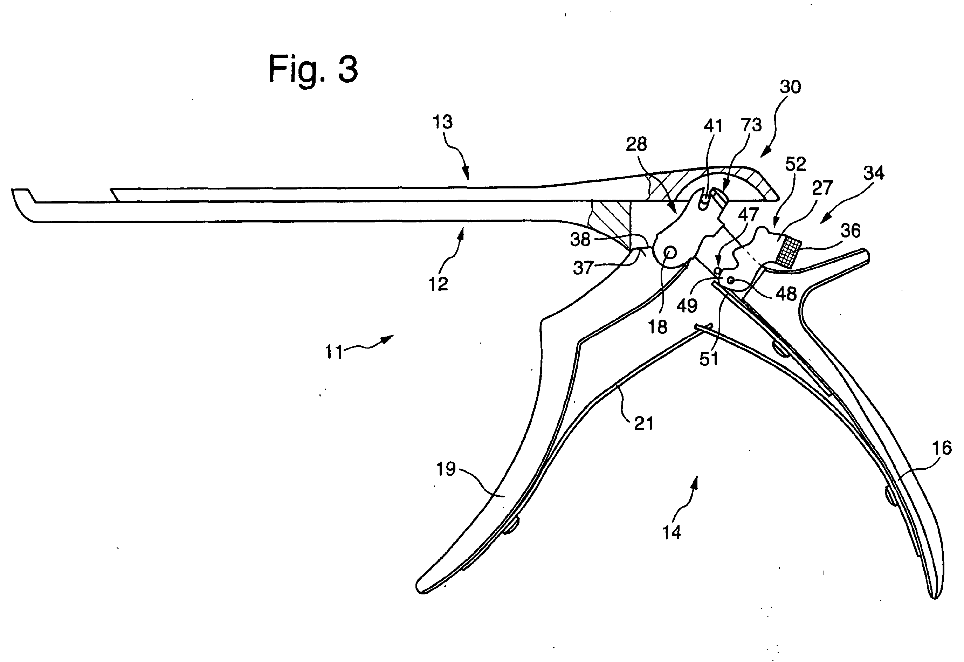

Zur Reinigung und Desinfektion des chirurgischen Instrumentes 11 ist erforderlich, daß das bewegbare Teil 13 von dem Hauptteil 12 zumindest teilweise abgehoben wird, um auch die Zwischenräume zu reinigen und zu desinfizieren. Die Verriegelungsvorrichtung 26 wird aus ihrer Verriegelungsposition 33, wie dies in Figur 1 und 2 dargestellt ist, in eine Entriegelungsposition 34 gemäß Figur 3 bis 6 übergeführt. Gemäß der Ausführungsform in den Figuren 1 bis 6 erfolgt dies durch Schwenken des Riegels 27, wobei vorteilhafterweise der betätigbare Handgriff 19 zumindest teilweise auf den feststehenden Handgriff 16 zubewegt ist, so daß der Riegel 27 mittels der Grifffläche 36 in die Entriegelungsposition 34 geschwenkt werden kann. Dadurch wird für den bewegbaren Teil 13 eine weitere Wegstrecke freigegeben, so daß dieser gegenüber dem Hauptteil 12 derart verschiebbar ist, daß die Führungselemente 31, 32 sich gegenseitig freigeben. Dieser Verschiebeweg wird durch einen an dem betätigbaren Handgriff 19 vorgesehenen Anschlag 37 begrenzt, der mit einer entsprechenden Fläche 38 im Übergangsbereich 17 zusammenwirkt.

Der bewegbare Teil 13 und der betätigbare Griff 19 sind vorteilhafterweise über ein lösbares Gelenk 41 wirkungsverbunden. Dieses Gelenk 41 ist schwenkbar ausgebildet, wodurch gleichzeitig eine Längsbewegung der Schwenkachse ermöglicht ist, um die kreisbogenförmige Bewegung des Hebelabschnittes 28 um die Drehachse 18 in eine Längsbewegung des bewegbaren Teils 13 entlang dem Hauptteil 12 zu ermöglichen.

In Figur 3 ist eine Zwischenposition 30 dargestellt, welche beim Überführen des chirurgischen Instrumentes 11 aus einer Ausgangsposition 22 gemäß Figur 1 in eine Position 39, 40 und 46 gemäß Figur 4, 5 oder 6 durchlaufen wird. Nach dem Entriegeln des Riegels 27 wird das bewegbare Teil 13 in die Zwischenposition 30 gemäß Figur 3 übergeführt, um anschließend durch Schwenken das bewegbare Teil 13 um das Gelenk 41 in eine erste Position 39 gemäß Figur 4 überzuführen. Dies kann durch einfaches Betätigen oder Drücken des Endabschnittes nach der Verbindung 41 erfolgen, um das bewegbare Teil 13 von dem Hauptteil 12 abzuheben. Diese Anordnung weist den Vorteil auf, daß die Zwischenräume zwischen dem bewegbaren Teil 13 und dem Hauptteil 12 gut zugänglich sind und darüber hinaus die Bauteile des chirurgischen Elementes miteinander gekoppelt sind.

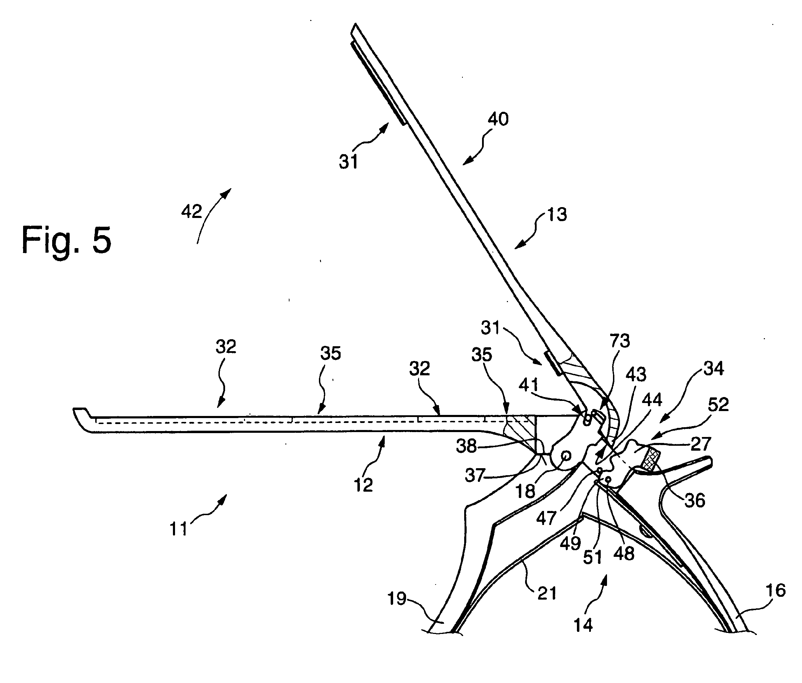

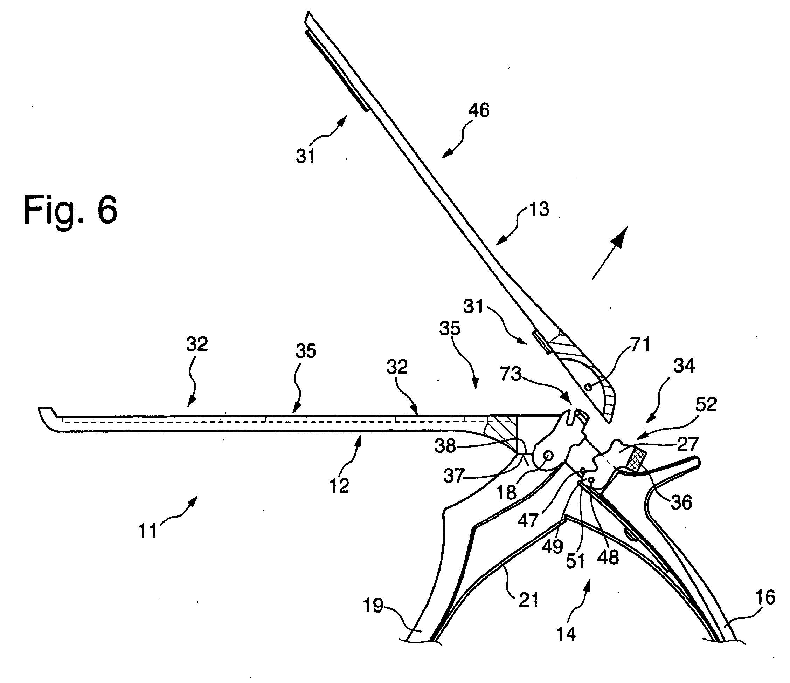

In Figur 5 ist das bewegbare Teil 13 in einer zweiten Position 40 dargestellt, welche erreicht wird, wenn das bewegbare Teil um die Gelenkverbindung 41 weiter in Pfeilrichtung 42 geschwenkt wird. Durch diese Schwenkbewegung wird eine zweite Phase eingeleitet, die dazu führt, daß das bewegbare Teil 13 vollständig zum Hauptteil 12 lösbar ist, wie aus Figur 6 hervorgeht. Während der Beibehaltung der Schwenkbewegung gemäß Pfeil 42 liegt ein zum feststehenden Griff 16 weisendes Ende 43 an einem Anschlag 44 an, welcher beispielsweise durch eine Körperlinie von dem betätigbaren Handgriff 16 im Übergang zur Auflagefläche des Hauptteils 12 ausgebildet ist. Dieser Anschlag 44 dient als Widerlager, so daß durch eine weitere Schwenkbewegung gemäß Pfeilrichtung 42 aufgrund der Hebelwirkung ein erleichtertes Lösen des beweglichen Bauteils 13 von dem Hauptteil 12 ermöglicht ist, wie in Fig. 6 dargestellt ist.

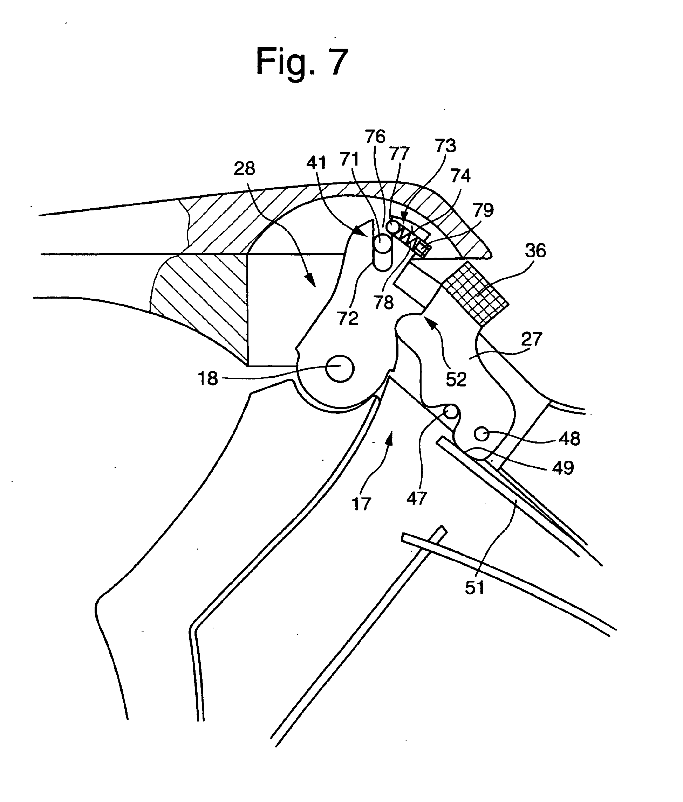

Eine erste Ausführungsform der lösbaren Gelenkverbindung 41 ist in Figur 7 dargestellt. Die lösbare Gelenkverbindung 41 umfaßt einen Stift 71, der an dem bewegbaren Teil 13 angeordnet ist sowie eine U-förmige Ausnehmung 72, welche in dem Hebelabschnitt 28 des betätigbaren Handgriffs 19 angeordnet ist. Des weiteren ist ein Rastelement 73 vorgesehen, welches bei Aufbringung einer Kraft durch den Stift 71 in eine Bohrung 74 des Hebelabschnitts 28 zurückgedrängt wird, damit der Stift 71 die Verengung 76, welche durch das Rastelement 73 gebildet ist, passieren kann. Das Rastelement 73 ist beispielsweise durch ein federnd nachgiebiges Rastelement ausgebildet, welches eine Kugel 77 aufweist, die in der Bohrung 74 positioniert ist. Eine zur U-förmigen Aufnahme 72 weisende Öffnung der Bohrung 74 ist kleiner als der Durchmesser der Kugel ausgebildet, so daß die Kugel nicht austreten kann. In der Bohrung 74 ist des weiteren ein Federelement 78 vorgesehen, welches durch einen Gewindestift 79 unter Vorspannung gehalten ist. Durch diesen Gewindestift 79 kann die Gelenkverbindung 41 hinsichtlich der benötigten Kraft für die Lösung der Verbindung 41 eingestellt werden. Anstelle des Gewindeabschnitts 79 kann auch ein eingepreßter Stift vorgesehen sein, wodurch eine einmalige Einstellung der Vorspannkraft ermöglicht ist. Alternativ zur Kugel 77 können auch weitere geometrischen Elemente eingebracht werden, die eine Verengung 76 der U-förmigen Aufnahme 72 bilden und in die Bohrung 74 zurückdrängbar sind.

Zum Reinigen des chirurgischen Instrumentes oder für den Austausch des bewegbaren Teils 13 oder des Hauptteils 12 kann die Gelenkverbindung gelöst werden.

Eine alternative Ausgestaltung der lösbaren Gelenkverbindung gemäß Figur 7 kann darin bestehen, daß die Verengung 76 durch ein Elastomer gebildet ist, welches im Bereich der U-förmigen Ausnehmung 72 eingesetzt, aufgeklebt, eingeklipst oder in sonstiger Weise befestigt ist. Anstelle eines Elastomers kann auch ein weiterer im elastischen Bereich verformbarer Kunststoff gegeben sein, der die Voraussetzung für die Sterilisation der chirurgischen Instrumente erfüllt.

Als weitere alternative Ausführungsform der lösbaren Gelenkverbindung kann vorgesehen sein, daß die U-förmige Ausnehmung 72 eine starre Verengung 76 aufweist und an dem Stift 71 eine Abflachung vorgesehen ist, welche in einer bestimmten Winkelposition ermöglicht, daß der Stift 71 an der Verengung 76 vorbeigeführt werden kann.

Eine weitere alternative Ausgestaltung der lösbaren Gelenkverbindung sieht vor, daß der Stift des bewegbaren Teils quer zur Hauptbewegungsrichtung des bewegbaren Teils von einer Arbeitsposition in eine Ausgangsposition und umgekehrt bewegbar ist, wobei dieser Stift wiederum eine Abflachung aufweist. In einer Position, die beispielsweise durch Drücken des Stiftes erzielt wird, kann die Abflachung in den Bereich der Verengung übergeführt werden, so daß der Stift an der Verengung vorbeigeführt wird und das bewegbare Teil abnehmbar ist. Diese Position, in welche der Stift durch Betätigung mit der Hand überzuführen ist, kann durch ein Federelement gesichert sein, so daß durch aktives Betätigen des Stiftes das bewegbare Teil von dem Hauptteil getrennt werden kann.

Für den Zusammenbau ist von Vorteil, daß zuerst der Stift 71 in die Aufnahme 72 eingesetzt wird, um anschließend in der Folge gemäß der Figur 6 bis Figur 1 das chirurgische Instrument 11 in eine Ausgangsposition oder Arbeitsposition 22, 23 überzuführen.

Die Herstellung der Einsatzbereitschaft des chirurgischen Instrumentes 11 erfolgt in umgekehrter Reihenfolge zur Demontage. Nachfolgend wird das bewegbare Teil 13 auf das Hauptteil 12 zubewegt, wobei die Führungselemente 31 in eine Aufnahme 35 des Hauptteils 12 angreifen, welche dann in die Führungselemente 32 übergehen. Anschließend wird der betätigbare Griff 19 auf den feststehenden Griff 16 zubewegt, wodurch die Führungselemente 31 in die Führungselemente 32 eingreifen. Vorteilhafterweise ist ein erster Abschnitt der Führungselemente 32 schräg angeordnet, so daß das bewegbare Teil während der axialen Bewegung in Richtung Schneide 24 gleichzeitig nach unten auf das Hauptteil 12 zubewegt wird, so daß nahezu ein spaltfreier Übergang zwischen dem bewegbaren Teil 13 und dem Hauptteil 12 in der Ausgangsposition vorgesehen ist. Der Riegel 27 wird in seine Verriegelungsposition 33 übergeführt, wobei dieser an einem Begrenzungselement 47 anliegt. Dadurch kann der Riegel 27 in eine definierte Position übergeführt werden, welche gleichzeitig die Ausgangsposition 22 bestimmt, da das Federelement 21 den betätigbaren Handgriff um die Drehachse 18 bewegt, wodurch der Hebelabschnitt 28 an dem Riegel 27 anliegt.

Der Riegel 27 ist um eine Achse 48 schwenkbar angeordnet. Nahe der Achse 48 ist eine Nase 49 vorgesehen, welche mit einer Feder 51 zusammenwirkt. Diese Feder 51 bewirkt zum einen, daß während der Betätigung des chirurgischen Instrumentes 11 der Riegel 27 in seiner Verriegelungsposition 33 als auch während der Reinigung in einer Entriegelungsposition 34 gehalten wird. Die Nase 49 ist dementsprechend ausgebildet, daß beide Endpositionen fixierbar sind.

Der Riegel 27 weist des weiteren einen Anlageabschnitt 52 auf, der sich aus einem geradlinigen Abschnitt und einem halbkreisförmigen Abschnitt zusammensetzt. Diese Ausgestaltung kann variabel ausgebildet sein, wobei die Funktion erfüllt sein muß, wonach bei der Bewegung des betätigbaren Griffes 19 aus der Arbeitsposition 29 in eine Ausgangsposition 22 der Hebelabschnitt 28 derart auf den Riegel 27 einwirkt, daß dieser gegen das Begrenzungselement 47 bewegt wird. Dadurch kann sichergestellt werden, daß nach jeden Arbeitshub das Verriegelungselement bei gegebenenfalls sich teilweise lösenden wieder in die Verriegelungsposition 33 zurückgeführt wird.

Alternativ zur Ausführungsform gemäß den Figuren 1 bis 7 kann vorgesehen sein, daß der feststehende Griff 16 und der betätigbare Griff 19 vertauscht sind. In Analogie kann hierzu auch der Verriegelungsmechanismus angeordnet sein. Ebenso ist auch denkbar, daß anstelle eines Riegels 27, der die Ausgangsposition 22 aufgrund einer Druckbelastung hält, diese auch durch eine Zugbelastung hält, beispielsweise dann, wenn das Verriegelungselement spiegelbildlich zu einer Linie angeordnet ist, welche beispielsweise zwischen der Drehachse 18 und dem Gelenk 41 gebildet wäre.

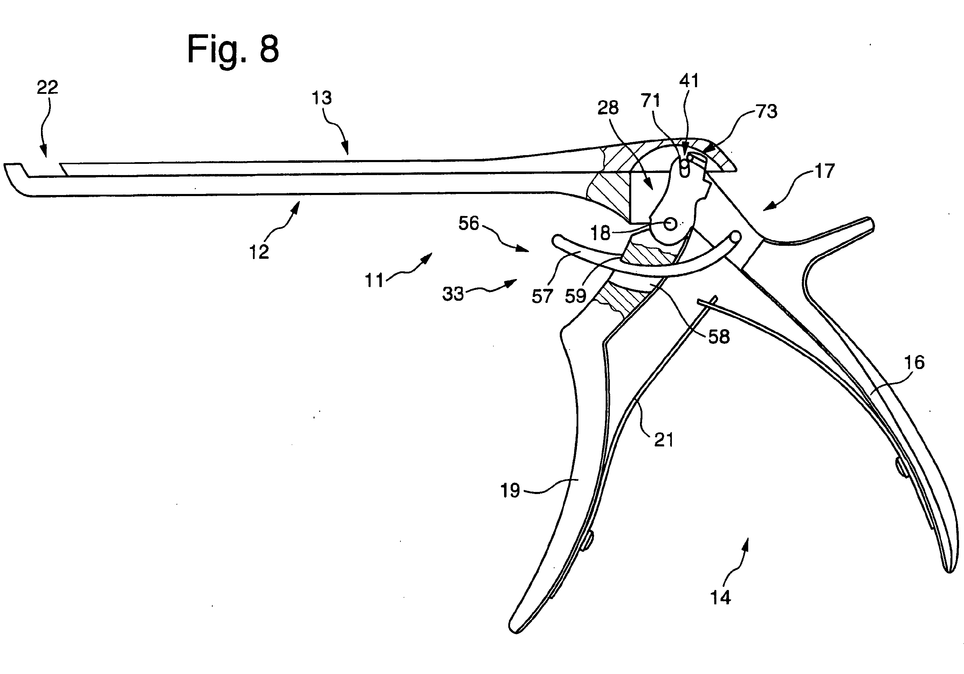

In Figur 8 ist eine alternative Ausführungsform einer Verriegelungsvorrichtung 56 dargestellt. Ein Riegel 57 ist schwenkbar an dem feststehenden Handgriff 16 angeordnet und kreuzt den betätigbaren Griff 19. Dabei ist vorteilhafterweise vorgesehen, daß in dem Griff 19 eine Ausnehmung 58 vorgesehen ist, in welcher der Riegel 57 geführt ist. Dieser weist einen Absatz 59 auf, durch welchen der bewegbare Teil 13 zum Hauptteil 12 in einer Ausgangsposition 22 anordenbar ist. Durch Drücken des Riegels 57 kann dieser Verriegelung gelöst werden, so daß für den Griff 19 ein weiterer Schwenkbereich freigegeben wird, um das bewegbare Teil 13 in eine Zwischenposition 30 gemäß Figur 3 überzuführen, welche anschließend in eine Position 39, 40 oder 46 gemäß Figur 4 bis 6 geschwenkt werden kann. Der erweiterte Schwenkbereich des Griffes 19 kann einerseits durch einen Anschlag 37 an einer Fläche 38 des Hauptteils 12 begrenzt sein oder durch eine weitere Rastnase, welche an dem Riegel 57 vorgesehen ist. Der Riegel 57 wird durch eine nicht näher dargestellte Feder in Richtung auf die Drehachse 18 gedrückt oder gezogen und ist während der Betätigung des chirurgischen Instruments 11 nachgiebig, so daß der Handgriff 19 schwenkbar zum feststehenden Griff 16 vorgesehen ist.

Alternativ kann vorgesehen sein, daß die Rastnase auch an einem unteren Abschnitt der Ausnehmung 58 angreift. Um das chirurgische Instrument 11 von einer Ausgangsposition 22 in eine Reinigungsposition 46 überzuführen, ist hierbei erforderlich, daß der Riegel 57 nach oben bewegt wird.

Es versteht sich, daß diese Anordnung auch spiegelbildlich vorgesehen sein kann, sowohl hinsichtlich der Anordnung des Riegels als auch hinsichtlich der Griffe 16, 19. Dies gilt auch für die weiteren Ausführungsformen.

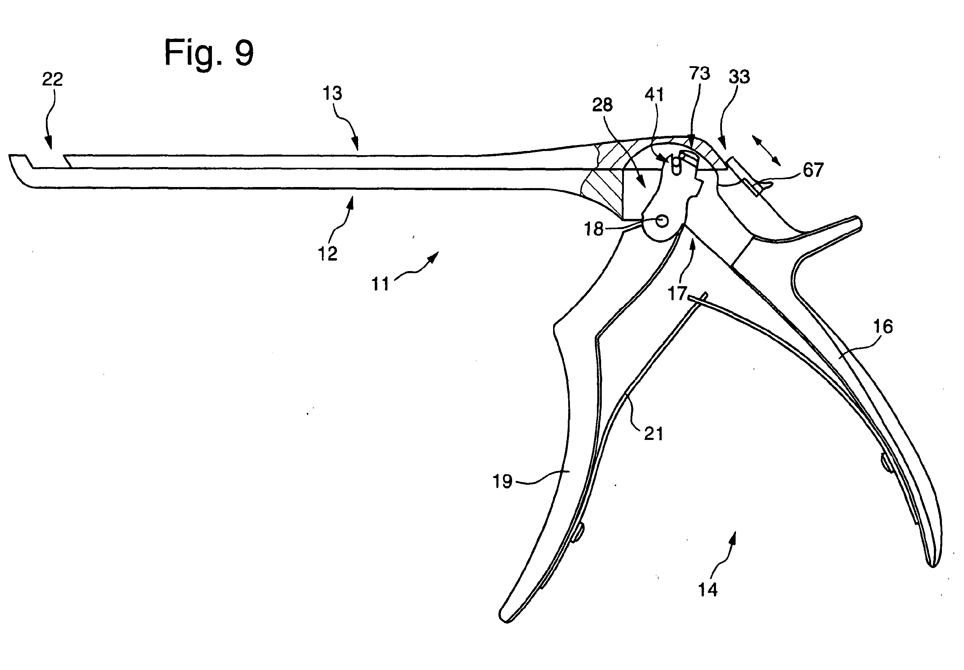

In Figur 9 ist eine weitere alternative Verriegelungsvorrichtung 66 dargestellt. Diese Verriegelungsvorrichtung 66 weist einen Riegel 67 auf, der an einem hinteren Ende des bewegbaren Teil 13 angreift und die Bewegung entlang dem Hauptteil 12 begrenzt. Durch eine Schiebebewegung zum freien Ende des Griffes 16 oder durch eine Schwenkbewegung um eine Drehachse, welche in jede Richtung denkbar ist, kann der Riegel 67 aus einer Verriegelungsposition 33, wie in Figur 6 dargestellt ist, in eine nicht näher dargestellte Entriegelungsposition übergeführt werden. Dadurch kann das bewegbare Teil 13 aus der in Figur 9 dargestellten Arbeitsposition 22 in die in Figur 3 dargestellte Zwischenposition 30 übergeführt werden. Ebenso kann vorgesehen sein, daß der Riegel 67 anstelle eines gemäß der Zeichnung dargestellten Anschlages auch unmittelbar an dem Hebelabschnitt 28, beispielsweise nahe des Gelenkes 41, angreift. Der Riegel 67 kann ebenso an dem bewegten Teil 13 vorgesehen sein und mit dem Handgriff 14 oder dem Hauptteil 12 zusammenwirken.

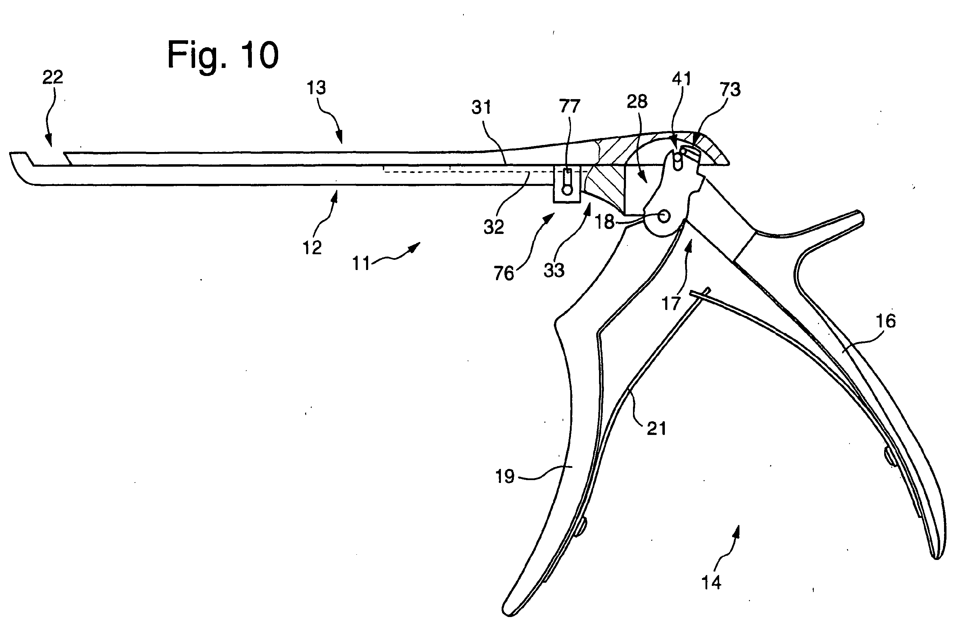

In Figur 10 ist eine weitere alternative Ausführungsform einer Verriegelungsvorrichtung 76 dargestellt. Diese Verriegelungsvorrichtung 76 ist an dem Hauptteil 12 vorgesehen und greift in das Führungselement 32 oder eine Aufnahme 35 ein. In einer Verriegelungsposition 33 sperrt diese Verriegelungsvorrichtung 76 durch einen Riegel 77 die Bewegung des bewegbaren Teiles 13 nach rechts im Sinne der Darstellung beziehungsweise in eine Ausgangsposition 22. Durch Drücken, Ziehen, Schieben oder Klappen um eine Drehachse kann der Riegel 77 betätigt werden und das Führungselement 32 freigeben, so daß das Führungselement 31 aus dem Führungselement 32 herausgeführt werden kann.

Sämtliche Ausführungsformen haben gemeinsam, daß das bewegliche Teil 13 gegenüber dem Hauptteil 12 bewegbar und abnehmbar anordenbar sind, wobei über eine lösbare Verbindung das bewegbare Teil zum Hauptteil oder Handgriff nach Überwinden einer vorbestimmten Kraft oder bei einer vorbestimmten Position der beiden Teile zueinander vorgesehen sind. Die Verriegelungsvorrichtung kann in Abhängigkeit der Ausführungsform an dem feststehenden Griff 16, dem betätigbaren Griff 19, dem Hauptteil 12 oder dem bewegbaren Teil 13 vorgesehen sein und an zumindest jeweils einem benachbarten Teil oder Griff in einer Verriegelungsposition angreifen. Die Verriegelungsvorrichtung kann durch Ziehen, Schieben, Drücken, Klappen, Schwenken oder dergleichen eines Riegels entriegelt und verriegelt werden. Es versteht sich, daß die entsprechenden, für chirurgische Instrumente geeigneten Materialien eingesetzt werden.

Alternativ zu den in den Figuren 1 bis 10 dargestellten chirurgischen Elementen, welche als sogenannte obenschneidende Stanzen ausgebildet sind, können auch unten-schneidende Stanzen vorgesehen sein. Die Schneiden 23, 24 sind spiegelbildlich zur Führungsebene des beweglichen Teils 13 am Hauptteil 12 vorgesehen. Diese Ausführungsformen können ebenso erfindungsgemäß ausgebildet sein. Das bewegbare Teil 13 ist dabei stufenförmig ausgebildet, wobei ein erster Abschnitt im Bereich der Verbindung 41 beibehalten ist und ein zur Schneide 23 führender Abschnitt auf der Unterseite des Hauptteils 12 verläuft. Damit dieselbe erfindungsgemäße Ausgestaltung ermöglicht und die daraus resultierenden Vorteile erzielt werden können, ist vorgesehen, daß in dem stufenförmigen Übergangsbereich von der Oberseite zur Unterseite ein Gelenk vorgesehen ist, wodurch der vordere stufenförmig abgesetzte Abschnitt bei einer Positionierung des bewegbaren Teils in einer Zwischenstation gemäß Figur 3 gegenüber dem Hauptteil 12 wegschwenkbar ist, um anschließend gemäß Figur 4 oder 5 schwenkbar oder gemäß Figur 6 lösbar zu sein. Derartige Abwandlungen oder ergänzende Maßnahmen, um von den erfindungsgemäßen Vorteilen Gebrauch zu machen, sind ebenfalls erfindungsgemäß umfaßt.

## Patentansprüche

1. Chirurgisches Instrument mit einem Hauptteil (12) und zumindest einem relativ dazu bewegbaren Teil (13), das mit einer daran angeordneten Führung (31) in einer komplementären Führung (32) des Hauptteils (12) geführt ist, mit einem an dem Hauptteil (12) angeordneten Handgriff (14), der einen feststehenden Griff (16) und einen das bewegbare Teil (13) betätigbaren Griff (19) aufweist, mit einer Verriegelungsvorrichtung (26, 56, 66, 76), die in einer ersten Position (33) das bewegbare Teil (13) in einer Arbeitsposition (29) und in einer Ausgangsposition (22) anordnet und in einer zweiten Position (34) das bewegbare Teil (13) von dem Hauptteil (12) zumindest teilweise abhebbar vorsieht, **dadurch gekennzeichnet, dass** zwischen dem bewegbaren Teil (13) und dem betätigbaren Griff (19) eine lösbare Gelenkverbindung (41) vorgesehen ist, welche in einer ersten Phase, nachdem das bewegbare Teil (13) bei Überführung aus der Ausgangsposition (22) in eine Position (39) aus der Führung (31, 32) freigekommen ist, das bewegbare Teil (13) und das Hauptteil (12) in der freien Position (39) miteinander koppelt und in einer zweiten Phase bewirkt, daß das bewegbare Teil (13) zum Hauptteil (12) zum vollständigen Abnehmen lösbar ist.

2. Chirurgisches Instrument nach Anspruch 1, **dadurch gekennzeichnet, dass** in der ersten Phase nach dem Lösen der Führung (31) des bewegbaren Teils (13) aus der Führung (32) des Hauptteils (12) das bewegbare Teil (13) um eine Schwenkachse der Gelenkverbindung (41) zumindest teilweise schwenkbar ist.

3. Chirurgisches Instrument nach einem der vorhergehenden Ansprüche, **dadurch gekennzeichnet, dass** die lösbare Gelenkverbindung (41) durch eine vorzugsweise U-förmige Aufnahme (72) in dem betätigbaren Handgriff (19) und einem darin gelagerten Stift (71) des bewegbaren Teils (13) ausgebildet ist und dass das offene Ende der Ausnehmung (72) durch ein federnd nachgiebiges Rastelement (73) verengt ist und vorzugsweise das Rastelement (73) eine Kugel (72) oder dergleichen aufweist, welches unter einer Federkraft in einer Bohrung (74) angeordnet ist und eine Verengung (76) der Aufnahme (72), die insbesondere U-förmig ausgebildet ist, bildet.

4. Chirurgisches Instrument nach Anspruch 3, **dadurch gekennzeichnet, dass** die Haltekraft des Rastelementes (73) einstellbar ist, vorzugsweise durch einen in der Bohrung (74) der Kugel (77) anordenbaren Gewindestift (79), der über ein Federelement (78) auf die Kugel (77) wirkt.

5. Chirurgisches Instrument nach einem der Ansprüche 1 oder 2, **dadurch gekennzeichnet, dass** die lösbare Gelenkverbindung (41) durch eine Aufnahme (72), vorzugsweise U-förmig, in dem betätigbaren Handgriff (19) und einem darin gelagerten Stift (71) des bewegbaren Teils (13) ausgebildet ist und dass das offene Ende der Aufnahme (72) durch ein elastisch nachgiebiges Rastelement, vorzugsweise einem Elastomer, ausgebildet ist.

6. Chirurgisches Instrument nach einem der Ansprüche 1 oder 2, **dadurch gekennzeichnet, dass** die lösbare Gelenkverbindung (41) durch eine Aufnahme (72), vorzugsweise U-förmig, in dem betätigbaren Handgriff (19) und einem darin gelagerten Stift (71) des bewegbaren Teils (13) ausgebildet ist und dass das offene Ende der Aufnahme (72) eine starre Verengung (76) aufweist und der in der Aufnahme (72) gelagerte Stift (71) eine Abflachung aufweist, welche in einer bestimmten Winkelposition kleiner als die lichte Weite der Verengung (76) ausgebildet ist.

7. Chirurgisches Instrument nach einem der Ansprüche 1 oder 2, **dadurch gekennzeichnet, dass** die lösbare Gelenkverbindung (41) durch eine Aufnahme, vorzugsweise U-förmig, in dem betätigbaren Handgriff (19) und einen darin gelagerten Stift (71) des bewegbaren Teils (13) ausgebildet ist und das offene Ende der Aufnahme (72) eine starre Verengung aufweist, wobei quer zur Öffnungs- und Schließbewegung des bewegbaren Teils der Stift (71) federgelagert ausgebildet ist, welche in einer ersten Position einen Durchmesser aufweist, der größer als die lichte Weite der Verengung (76) ist und in einer weiteren betätigbaren Position einen Durchmesser aufweist, der kleiner als die lichte Weite der Verengung (76) ausgebildet ist.

8. Chirurgisches Instrument nach einem der vorhergehenden Ansprüche, **dadurch gekennzeichnet, dass** beim Überführen des bewegbaren Teils (13) aus einer Reinigungsposition (46) in die Arbeitsposition (22) die Führungsabschnitte (31) des bewegbaren Teils (13) in Aufnahmen des Hauptteils (12) eingreifen und durch Drücken des betätigbaren Griffs (19) die Führungen (31, 32) selbsttätig ineinander greifen und vorzugsweise die Führung (32) im Hauptteil (12) einen ersten schrägverlaufenden Abschnitt aufweist, welcher das bewegbare Teil (13) auf das Hauptteil (12) zubewegt und in eine Arbeitsposition (22) überführt.

9. Chirurgisches Instrument nach einem der vorhergehenden Ansprüche, **dadurch gekennzeichnet, dass** die Verriegelungsvorrichtung (26, 56, 67, 77) einen Riegel (27, 57, 67, 77) aufweist, der kraft- und/oder formschlüssig zumindest in einer Verriegelungsposition (33) anordenbar ist.

10. Chirurgisches Instrument nach einem der vorhergehenden Ansprüche, **dadurch gekennzeichnet, dass** die Verriegelungsvorrichtung (26, 56, 66) einen Riegel (27, 57, 67) aufweist, der an dem Handgriff (14) vorgesehen ist und vorzugsweise der Riegel (27, 57, 67) an dem feststehenden oder betätigbaren Griff (16, 19) vorgesehen ist.

11. Chirurgisches Instrument nach einem der vorhergehenden Ansprüche, **dadurch gekennzeichnet, dass** an dem feststehenden Griff (19) ein schwenkbarer Riegel (27) vorgesehen ist, der nahe einer Schwenkachse (18) an dem betätigbaren Griff (19) angreift und vorzugsweise der schwenkbare Riegel (27) einen seine Verriegelungsposition (33) bestimmendes Begrenzungselement (47) aufweist.

12. Chirurgisches Instrument nach einem der vorhergehenden Ansprüche 9 bis 11, **dadurch gekennzeichnet, dass** der Riegel (27) in einer Entriegelungsposition (34) schwenkbar einen weiteren Schwenkbereich des betätigbaren Griffs (19) freigibt, welcher von einem an dem Hauptteil (12) vorgesehenen Anschlag (37) begrenzt ist und vorzugsweise der Riegel (27) an einem Hebelabschnitt (28) zwischen der Schwenkachse (18) und dem Gelenk (41) angreift.

13. Chirurgisches Instrument nach einem der vorhergehenden Ansprüche 9 bis 12, **dadurch gekennzeichnet, dass** der Riegel (27) einen Verriegelungsabschnitt aufweist, der an einem komplementär ausgebildeten Hebelabschnitt (28) des betätigbaren Griffs (19) angreift und vorzugsweise der Verriegelungsabschnitt eine Hinterschneidung aufweist, die insbesondere durch eine Schräge und ein sich daran anschließenden Rastnocken ausgebildet ist.

14. Chirurgisches Instrument nach einem der Ansprüche 1 bis 11, **dadurch gekennzeichnet, dass** der Riegel (57) an einem dem Hebelabschnitt (28) gegenüberliegenden Abschnitt des betätigbaren Griffs (19) angreift und vorzugsweise der Riegel (57) in einer Ausnehmung (58) des betätigbaren Griffs (19) geführt ist.

15. Chirurgisches Instrument nach Anspruch 14, **dadurch gekennzeichnet, dass** an dem Riegel (57) ein Absatz (59) vorgesehen ist, der den betätigbaren Griff (19) in einer Ausgangsposition (22) positioniert.

16. Chirurgisches Instrument nach einem der Ansprüche 14 oder 15, **dadurch gekennzeichnet, dass** durch Lösen der Vorrichtung zwischen dem Absatz (59) dem Riegel (57) und einem Randbereich der Ausnehmung (58) ein weiterer Schwenkbereich des betätigbaren Griffs (19) freigegeben wird und der betätigbare Griff (19) bis zum Anschlag (37) am Hauptteil (12) oder einem weiteren Absatz des Riegels (57) schwenkbar ist.

17. Chirurgisches Instrument nach einem der Ansprüche 14 bis 16, **dadurch gekennzeichnet, dass** beim Überführen des betätigbaren Griffs (19) aus einer Reinigungsposition (46) in eine Arbeitsposition (22) eine selbständige Verriegelung des Riegels (57) vorgesehen ist und vorzugsweise der betätigbare Griff (19) in einer Ausgangsposition angeordnet ist.

18. Chirurgisches Instrument nach einem der Ansprüche 1 bis 14, **dadurch gekennzeichnet, dass** am feststehenden Griff (16) ein Riegel (67) vorgesehen ist, der an dem beweglichen Teil (13), vorzugsweise an einem Endabschnitt des beweglichen Teils (13), angreift und vorzugsweise der Riegel (67) schwenkbar oder verschiebbar ausgebildet ist.

19. Chirurgisches Instrument nach Anspruch 18, **dadurch gekennzeichnet, dass** der Riegel (67) in einer Verriegelungsposition (33), vorzugsweise durch eine lösbare Rastverbindung, gesichert ist.

20. Chirurgisches Instrument nach einem der Ansprüche 1 bis 11, **dadurch gekennzeichnet, dass** die Verriegelungsvorrichtung (76) einen Riegel (77) aufweist, der an dem Hauptteil (12) oder bewegbaren Teil (13) vorgesehen ist.

21. Chirurgisches Instrument nach Anspruch 20, **dadurch gekennzeichnet, dass** die an dem Hauptteil (12) vorgesehene Verriegelungsvorrichtung (76) einen Riegel (77) aufweist, der in eine Führung (32) im Hauptteil (12) eingreift, in welcher der bewegbare Teil (13) zumindest abschnittsweise geführt ist und vorzugsweise der Riegel (77) an einem den Arbeitshub des bewegbaren Teils (13) begrenzenden Bereich vorgesehen ist.

22. Chirurgisches Instrument nach einem der Ansprüche 20 oder 21, **dadurch gekennzeichnet, dass** der Riegel (77) durch einen Druck-, Zug-Schiebe-Mechanismus lösbar ist.

## Claims

1. Surgical instrument with a main part (12) and at least one part (13) movable relative thereto which is guided with a guide (31) arranged thereon in a complementary guide (32) of the main part (12), with a handle (14) arranged on the main part (12) and having a stationary handle (16) and a handle (19) which can actuate the movable part (13), with a locking device (26, 56, 66, 76) providing in a first position (33) the movable part (13) in a working position (29) and in an initial position (22) and providing in a second position (34) the movable part (13) removably at least partially from the main part (12), **characterized in that** a releasable articulated connection (41) is provided between the movable part (13) and the actuatable handle (19), which in a first phase after the movable part (13) has come free from the guide (31, 32) on transfer from an initial position (22) into a position (39) connects the movable part (13) and the main part (12) with one another in the free position (39), and causes in a second phase that the movable part (13) is completely removable from the main part (12).

2. Surgical instrument according to claim 1, **characterized in that** in the first phase after the release of the guide (31) of the movable part (13) from the guide (32) of the main part (12), the movable part (13) is at least partially pivotable around a pivot axis of the articulated connection (41).

3. Surgical instrument according to one of the preceding claims, **characterized in that** the releasable articulated connection (41) is constituted by a preferably U-shaped seating (72) in the actuatable handle (19) and a pin (71) mounted therein of the movable part (13), and wherein the open end of the recess (72) is constricted by a resiliently yieldable latch element (73) and preferably the latch element (73) has a ball (72) or the like which is arranged under a spring force in a bore (74) and a forms constriction (76) of the seating (72) which is preferably constituted in a U-shape.

4. Surgical instrument according to claim 3, **characterized in that** the holding force of the latch element (73) is adjustable, preferably by means of a threaded pin (79) which can be arranged in the bore (74) of the ball (77) and which acts on the ball (77) by means of a spring element (78).

5. Surgical instrument according to one of claims 1 or 2, **characterized in that** the releasable articulated connection (41) is constituted by a seating (72), preferably U-shaped, in the actuatable handle (19) and a pin (71) mounted therein of the movable part (13), and wherein the open end of the seating (72) is constituted by an elastically yieldable latch element, preferably an elastomer.

6. Surgical instrument according to one of claims 1 or 2, **characterized in that** the releasable articulated connection (41) is constituted by a seating (72), preferably U-shaped, in the actuatable handle (19), and a therein mounted pin (71) of the movable part (13), and wherein the open end of the seating (72) has a rigid constriction (76) and the pin (71) mounted in the seating (72) has a flat which in a given angular position is constituted smaller than the inside width of the constriction (76).

7. Surgical instrument according to one of claims 1 or 2, **characterized in that** the releasable articulated connection (41) is constituted by a seating, preferably U-shaped, in the actuatable handle (19), and a therein mounted pin (71) of the movable part (13), and wherein the open end of the seating (72) has a rigid constriction, and wherein the pin (71) is constituted spring-mounted transversely of the opening and closing movement of the movable part and has in a first position a diameter greater than the inside width of the constriction (76) and has in a further, actuatable, position, a diameter constituted smaller than the inside width of the constriction (76).

8. Surgical instrument according to one of the preceding claims, **characterized in that**, upon changing over the moved part (13) from a cleaning position (46) to the working position (22), the guide sections (31) of the movable part engage in seatings of the main part (12), and by means of pressing the actuatable handle portion (19) the guides (31, 32) automatically engage in each other and preferably the guide (32) has a first section which runs obliquely in the main part (12), and which moves the movable part (13) toward the main part (12) and changes it over into a working position (22).

9. Surgical instrument according to one of the preceding claims, **characterized in that** the locking device (26, 56, 67, 77) has a latch (27, 57, 67,77) which can be frictionally and/or positively disposed at least in a locking position (33).

10. Surgical instrument according to one of the preceding claims, **characterized in that** the locking device (26, 56, 66) has a latch (27, 57, 67) which is provided on the handle (14) and preferably the latch (27, 57, 67) is provided on the stationary or actuatable handle portion (16, 19).

11. Surgical instrument according to one of the preceding claims, **characterized in that** a pivotable latch (27) is provided on the stationary handle portion (19) and engages the actuatable handle portion (19) near a hinge pin (18) and preferably the pivotable latch (27) has a bounding element (47) determining its locking position (33).

12. Surgical instrument according to one of the claims 9 to 11, **characterized in that** the latch (27) in an unlocking position (34) pivotably releases a further pivoting region of the actuatable handle portion (19), which is limited by a stop (37) provided on the main part (12) and preferably the latch (27) engages on a lever section (28) between the hinge pin (18) and the articulation (41).

13. Surgical instrument according to one of the claims 9 to 12, **characterized in that** the latch (27) has a locking section which engages on a complementarily constituted lever section (28) of the actuatable handle portion (19) and preferably the locking section has an undercut, which is preferably formed by a bevel and a thereto adjoining detent cam.

14. Surgical instrument according to one of the claims 1 to 11, **characterized in that** the latch (57) engages a section of the actuatable handle portion (19) opposite the lever section (28) and preferably the latch (57) is guided in a recess (58) of the actuatable handle portion (19).

15. Surgical instrument according to claim 14, **characterized in that** a shoulder (59) is provided on the latch (57) and positions the actuatable handle portion (19) in an initial position (22).

16. Surgical instrument according to one of the claims 14 to 15, **characterized in that** by releasing the device between the shoulder (59), the latch (57), and an edge region of the recess (58), a further pivoting region of the actuatable handle portion (19) is released and the actuatable handle portion (19) is pivotable as far as the stop (37) on the main part (12) or a further stop of the latch (57).

17. Surgical instrument according to one of the claims 14 to 16, **characterized in that** on changing over the actuatable handle portion (19) from a cleaning position (46) into a working position (22) an automatic locking of the latch (57) is provided and preferably the actuatable handle portion (19) is arranged in an initial position.

18. Surgical instrument according to one of the claims 1 to 14, **characterized in that** a latch (67) is provided on the stationary handle portion (16) and engages on the movable part (13), preferably at an end section of the movable part (13) and preferably the latch (67) is constituted pivotably or displaceably.

19. Surgical instrument according to claim 18, **characterized in that** the latch (67) is secured in a locking position (33), preferably by means of a releasable detent connection.

20. Surgical instrument according to one of the claims 1 to 11, **characterized in that** the locking device (76) has a latch (77) which is provided on the main part (12) or on the movable part (13).

21. Surgical instrument according to claim 20, **characterized in that** the locking device (76) provided on the main part (12) has a latch (77) which engages in a guide (32) in the main part (12), in which guide the movable part (13) is guided at least sectionwise and preferably the latch (77) is provided on a region limiting the working stroke of the movable part (13).

22. Surgical instrument according to one of the claims 20 to 21, **characterized in that** the latch (77) is releasable by means of a pressing, pulling, or sliding mechanism.

## Revendications

1. Instrument chirurgical comportant une pièce principale (12) et au moins une pièce mobile (13) par rapport à celle-ci et guidée par un guide (31) disposé au niveau de la pièce principale (12) dans un guide complémentaire (32), un élément de préhension (14) disposé au niveau de la pièce principale (12) et qui comporte une poignée fixe (16) et une poignée (19) pouvant actionner la pièce mobile (13), un dispositif de verrouillage (26, 56, 66, 76) qui, dans une première position (33), met la pièce mobile (13) dans une position opérationnelle (29) et dans une position initiale (22) et, dans une deuxième position (34), permet que la pièce mobile (13) puisse du moins partiellement être relevée de la pièce principale (12), **caractérisé en ce qu'**entre la pièce mobile (13) et la poignée actionnable (19), il est prévu une jonction articulée dissociable (41) qui, dans un premier stade, une fois que la pièce mobile (13) s'est libérée en passant de la position initiale (22) à une position (39) hors du guide (31, 32), accouple l'une à l'autre la pièce mobile (13) et la pièce principale (12) en position libre (39) et, dans un deuxième stade, a pour effet que la pièce mobile (13) est dissociable de la pièce principale (12) pour permettre de l'enlever totalement.

2. Instrument chirurgical selon la revendication 1, **caractérisé en ce qu'**au premier stade, après dissociation du guide (31) de la pièce mobile (13) hors du guide (32) de la pièce principale (12), la pièce mobile (13) peut pivoter du moins partiellement autour d'un axe de pivotement de la jonction articulée (41).

3. Instrument chirurgical selon l'une quelconque des revendications précédentes, **caractérisé en ce que** la jonction articulée dissociable (41) est constituée par un support (72) de préférence en forme de U placé dans l'élément de préhension actionnable (19) et dans une tige (71) s'appuyant dedans de la pièce mobile (13) et **en ce que** l'extrémité ouverte de l'évidement (72) est rétrécie par un élément d'enclenchement à résilience de ressort (73) et l'élément d'enclenchement (73) comporte de préférence une bille (72) ou similaire qui est disposé sous une force de ressort dans un alésage (74) et constitue un rétrécissement (76) du support (72) réalisé notamment en forme de U.

4. Instrument chirurgical selon la revendication 3, **caractérisé en ce que** la force de retenue de l'élément d'enclenchement (73) est réglable, de préférence au moyen d'une tige filetée (79) pouvant être disposée dans l'alésage (74) de la bille (77) et agissant par le biais d'un élément à ressort (78) sur la bille (77).

5. Instrument chirurgical selon l'une quelconque des revendications 1 ou 2, **caractérisé en ce que** la jonction articulée dissociable (41) est constituée par un support (72) de préférence en forme de U placé dans l'élément de préhension actionnable (19) et dans une tige (71) s'appuyant dedans de la pièce mobile (13) et **en ce que** l'extrémité ouverte du support (72) est constituée par un élément d'enclenchement à résilience élastique, de préférence un élastomère.

6. Instrument chirurgical selon l'une quelconque des revendications 1 ou 2, **caractérisé en ce que** la jonction articulée dissociable (41) est constituée par un support (72) de préférence en forme de U placé dans l'élément de préhension actionnable (19) et dans une tige (71) s'appuyant dedans de la pièce mobile (13) et **en ce que** l'extrémité ouverte du support (72) présente un rétrécissement rigide (76) et que la tige (71) s'appuyant dans le support (72) présente un aplatissement qui, dans une certaine position angulaire, est plus petit que le diamètre intérieur du rétrécissement (76).

7. Instrument chirurgical selon l'une quelconque des revendications 1 ou 2, **caractérisé en ce que** la jonction articulée dissociable (41) est constituée par un support de préférence en forme de U placé dans l'élément de préhension actionnable (19) et dans une tige (71) s'appuyant dedans de la pièce mobile (13) et l'extrémité ouverte du support (72) présente un rétrécissement rigide, la tige (71) ayant, transversalement au mouvement d'ouverture et de fermeture de la pièce mobile, un palier à ressort présentant, dans une première position, un diamètre supérieur au diamètre intérieur du rétrécissement (76) et, dans une autre position actionnable, un diamètre inférieur au diamètre intérieur du rétrécissement (76).

8. Instrument chirurgical selon l'une quelconque des revendications précédentes, **caractérisé en ce que**, lors du passage de la pièce mobile (13) d'une position de nettoyage (46) à la position opérationnelle (22), les sections de guidage (31) de la pièce mobile (13) s'engrènent dans des supports de la pièce principale (12) et que, du fait de la pression de la poignée actionnable (19), les guides (31, 32) s'engrènent automatiquement l'un dans l'autre et que le guide (32) de la pièce principale (12) présente de préférence une première section s'étendant à l'oblique qui amène la pièce mobile (13) vers la pièce principale (12) et la fait passer en position opérationnelle (22).

9. Instrument chirurgical selon l'une quelconque des revendications précédentes, **caractérisé en ce que** le dispositif de verrouillage (26, 56, 67, 77) comporte un verrou (27, 57, 67, 77) qui peut être mis en correspondance mécanique et/ou géométrique du moins dans une position de verrouillage (33).

10. Instrument chirurgical selon l'une quelconque des revendications précédentes, **caractérisé en ce que** le dispositif de verrouillage (26, 56, 66) comporte un verrou (27, 57, 67) qui est prévu sur l'élément de préhension (14) et que le verrou (27, 57, 67) est prévu de préférence sur la poignée fixe ou actionnable (16, 19).

11. Instrument chirurgical selon l'une quelconque des revendications précédentes, **caractérisé en ce qu'**il est prévu sur la poignée fixe (16) un verrou pivotable (27) qui s'engrène à proximité d'un axe de pivotement (18) sur la poignée actionnable (19) et que le verrou pivotable (27) comporte de préférence un élément de limitation (47) définissant sa position de verrouillage (33).

12. Instrument chirurgical selon l'une quelconque des revendications précédentes 9 à 11, **caractérisé en ce que** le verrou (27), dans une position de déverrouillage (34), libère de manière pivotable une autre zone de pivotement de la poignée actionnable (19) qui est limitée par une butée (37) prévue sur la pièce principale (12) et que le verrou (27) s'engrène de préférence sur une section de levier (28) entre l'axe de pivotement (18) et l'articulation (41).

13. Instrument chirurgical selon l'une quelconque des revendications précédentes 9 à 12, **caractérisé en ce que** le verrou (27) comporte une section de verrouillage qui s'engrène sur une section de levier (28) constituée de manière complémentaire de la poignée actionnable (19) et que la section de verrouillage comporte de préférence une contre-dépouille qui est constituée notamment par un biseau et une came d'enclenchement s'y raccordant.

14. Instrument chirurgical selon l'une quelconque des revendications 1 à 11, **caractérisé en ce que** le verrou (57) s'engrène sur une section opposée à la section de levier (28) de la poignée actionnable (19) et que le verrou (57) est de préférence guidé dans un évidement (58) de la poignée actionnable (19).

15. Instrument chirurgical selon la revendication 14, **caractérisé en ce qu'**il est prévu sur le verrou (57) un talon (59) qui positionne la poignée actionnable (19) dans une position initiale (22).

16. Instrument chirurgical selon l'une quelconque des revendications 14 ou 15, **caractérisé en ce qu'**en dissociant le dispositif entre le talon (59), le verrou (57) et une zone périphérique de l'évidement (58), on libère une autre zone de pivotement de la poignée actionnable (19) et que la poignée actionnable (19) peut pivoter jusqu'à la butée (37) placée sur la pièce principale (12) ou sur un autre talon du verrou (57).

17. Instrument chirurgical selon l'une quelconque des revendications 14 à 16, **caractérisé en ce que**, lors du passage de la poignée actionnable (19) d'une position de nettoyage (46) à une position opérationnelle (22), il est prévu un verrouillage automatique du verrou (57) et que la poignée actionnable (19) est disposée de préférence dans une position initiale.

18. Instrument chirurgical selon l'une quelconque des revendications 1 à 14, **caractérisé en ce qu'**il est prévu sur la poignée fixe (16) un verrou (67) qui s'engrène sur la pièce mobile (13), de préférence sur une section terminale de la pièce mobile (13), et que le verrou (67) est réalisé de préférence de manière à pouvoir pivoter ou se déplacer.

19. Instrument chirurgical selon la revendication 18, **caractérisé en ce que** le verrou (67) est bloqué dans une position de verrouillage (33), de préférence par une jonction à enclenchement dissociable.

20. Instrument chirurgical selon l'une quelconque des revendications 1 à 11, **caractérisé en ce que** le dispositif de verrouillage (76) comporte un verrou (77) qui est prévu sur la pièce principale (12) ou sur la pièce mobile (13).

21. Instrument chirurgical selon la revendication 20, **caractérisé en ce que** le dispositif de verrouillage (76) prévu sur la pièce principale (12) comporte un verrou (77) qui s'engrène dans un guide (32) dans la pièce principale (12), guide dans lequel la pièce mobile (13) est guidée du moins par sections et que le verrou (77) est prévu de préférence dans une zone limitant la course opérationnelle de la pièce mobile (13).

22. Instrument chirurgical selon l'une quelconque des revendications 20 ou 21, **caractérisé en ce que** le verrou (77) peut être débloqué par un mécanisme de pression-traction-poussée.
